Europäisches Patentamt

European Patent Office

Office européen des brevets

(11) Publication number: **0 367 320**
**A1**

## (12) EUROPEAN PATENT APPLICATION

(21) Application number: 89202503.2

(51) Int. Cl.5: **A61N 1/04 , A61N 1/36**

(22) Date of filing: **04.10.89**

The title of the invention has been amended (Guidelines for Examination in the EPO, A-III, 7.3).

(30) Priority: **05.10.88 DK 5562/88**
**09.06.89 DK 2837/89**

(43) Date of publication of application:
**09.05.90 Bulletin 90/19**

(84) Designated Contracting States:
**AT BE CH DE ES FR GB GR IT LI LU NL SE**

(71) Applicant: **Kornerup, Niels**
**Gl. Valleroedvej 31**
**DK-2960 Rungsted(DK)**

(72) Inventor: **Kornerup, Niels**
**Gl. Valleroedvej 31**
**DK-2960 Rungsted(DK)**

(74) Representative: **de Bruijn, Leendert C. et al**
**Nederlandsch Octrooibureau**
**Scheveningseweg 82 P.O. Box 29720**
**NL-2502 LS 's-Gravenhage(NL)**

(54) **Electric generator, compress, combination of compresses, and system for the treatment of wounds by means of electric stimulation.**

(57) A wound treatment system comprises an electrically conductive wound compress (2), a return electrode (3), an electric generator (7) and cables (4) for forming electric connections between said components. According to the invention the wound compress comprises a laminate with a first layer (17) of electrically conductive material capable of forming an essentially uninterrupted surface contact with the wound surface and a second layer (16) of electrically conductive electrode material, said second layer exhibiting a conductivity in directions parallel to the planes of the layers which is higher than the corresponding value of the first layer, said second layer (16) being adapted for attachment of an electric connection, and said generator (7) being adapted to optionally emit a direct current signal or a pulsed signal comprising pulses with a pulse duration around 0,1 ms or a combination of the above signals.

Fig. 1

## BACKGROUND OF THE INVENTION

### Field of the Invention

The present invention concerns systems for the treatment of wounds by electric stimulation. The invention more particularly concerns an electric generator, a compress, a combination of compresses and a wound treatment system. The invention further concerns a method for the treatment of wounds.

### Description of Prior Art

Chronic wounds, which do not heal, represent a serious problem for a substantial proportion of the population. Many persons thus have experienced or heard about chronic leg sores or decubitus ulcers. Such wounds may be caused by age or by impaired mobility, but may also occur in relation with a number of other circumstances, e.g. diabetes, venous stasis, thrombangitis obliterans, arterio sclerosis, parkinsonism, congelation a.o. The causes are believed to be related to insufficient blod flow through the affected region. These wounds may be extremely painful and may grow to a considerable size, and more or less extensive gangrene may occur. In numerous cases it has been impossible to find any effective method of treatment, and the solution suggested to the patient is quite often amputation of the affected portions of the body, if possible. Experience shows that these conditions are generally followed by a deterioration of the general condition, which may ultimately lead to the death of the patient.

With the attempt to remedy these problems treatment with electric nerve stimulation has been tried in the prior art.

A first prior art method is treatment with short electric pulses applied in a continuous sequence with a repetition frequency around 100 Hz. These signals are applied through needle electrodes inserted in the skin or through electrodes applied externally onto the skin over a period of time lasting from 15 to 45 minutes. By this method it has been possible to achieve some pain-relief effect, placing the pair of electrodes on selected portions on a hand similarly as the portions of the hand used in acupuncture treatments, i.e. an implacement which is not adjacent the wound. This method is some times referred to as TENS (transcutane electric nerve stimulation). As mentioned, the effect is pain relieving and the method has not been able to prove any significant healing effect. The pain-relief effect persists for several hours after a period of application of electric signals.

A second method of treatment with electric nerve stimulation is described by Birger Kaada in the article, "Perifere sirkulationsforstyrrelser og kroniske sår", in the Norwegian periodical, Fysioterapeuten, vol. 51, February 1984, p. 80-89. This article describes treatment with rhythmic pulse signals, a train comprising five pulses being applied twice every second, each pulse train lasting 50 ms and being followed by 450 ms intermission. The duration of the individual pulses is 0,2 ms, and the pulse frequency within each pulse train is 100 Hz. The signal is applied through a pair of skin electrodes placed onto positions on the same hand. This method of treatment has in many cases been proved able to enhance the blood flow in all extremities, and good healing effect has been observed in many cases. The reason is believed to be related with the induction of rhythmic contractions of the musculature, in particular the musculature at the skin and around blood vessels near the skin. According to this article the period of stimulation lasts 30-35 minutes, and the effect in the form of the enhanced blood flow sustains over 4-8 hours. A number of problems has been encountered by practicing this method, i.e. skin irritation at the electrodes and induced headache, and the applicability of this method is therefore considered to be limited.

A method for wound treatment with electric stimulation directly at the wound region has been described by Patrick J. Carley and Stanley F. Wainapel in the article, "Electrotherapy for Acceleration of Wound Healing: Low Intensity Direct Current", in the periodical Arch Phys Med Rehabil, vol. 66, July 1985, p. 443-446. According to this method a direct current of low intensity, e.g. 30-110 $\mu$A per $cm^2$ is applied adjacent to or directly into the wound. The return electrode is spaced 15 to 25 cm from the wound. The wound is irrigated with salt water and covered by a salt damped gaze, an electrode being placed on top of this layer. The dressing is replaced daily. During one day electric stimulation is applied for two hours followed by a two to four hour intermission and thereafter again two hours of stimulation. The authors claim good results in the form of accelerated wound healing, suggesting, though, the occurrence of skin irritation at the electrodes. The electrode described is believed to have substantial disadvantages. The application of the electrodes must be laborious and bears the risk of causing pain. The electrode is believed to be sensitive to liquid which may occur by oozing wounds, involving the danger of loosening the electrode or changing the conductivity properties of the electrode. Changes in the conductivity properties may lead to undesirable changes of the stimulation signal, and in the case of more localized variations, there may be the risk that the current applied will be concentrated over more limited areas with the danger of developing hot spots, skin irritation or of shunting the current around the area where it should have been applied. In case the skin or the wound exhibits a non-planar surface, there may be risks of gaps where the gaze does not conform to the skin and where no current is applied. Another risk is that of a drying-out of the gaze, whereby the conductivity may drop unacceptably. A further disadvantage is the use of a reusable electrode involving risks of contamination and infection.

US patent no. 4 706 680 discloses an adhesive skin electrode developed for TENS (mentioned above), for recording electro cardiograms or for the application of electric defibrillation signals. This electrode comprises a viscous hydrophilic gel, which is described in details in the co-assigned US patent no. 4 684 558. The gel is used as contact element to the skin and a reusable electrode may be placed in contact with the distal side of the gel relative to the skin. This gel is manufactured from an aqueous solution of linear water-soluable polyethylene oxide by crosslinking the polyethylene oxide through radiation so as to develop a cohesive viscous gel. The gel can be made electrically conductive by the admixing before radiation of a dissolved electrolyte, e.g. a salt. A variety of conductivity values may be obtained by a suitable selection of a salt concentration.

## SUMMARY OF THE INVENTION

### Brief description

The present invention provides a wound treatment system comprising an electrically conductive wound compress, a return electrode, an electric generator and means, e.g. cables, for electrically interconnecting the above parts, which system is characterized in that the wound compress comprises a laminate with a first layer of electrically conductive material capable of forming an uninterrupted surface contact with the surface of a wound or with a skin surface, and a second layer of electrically conductive electrode material, said second layer exhibiting an electric conductivity in directions parallel to the planes of the layers which is higher than that of the first layer, said second layer being adapted for attachment of an electric connection, and in that the generator is adapted to emit either a direct current signal or a pulsed signal comprising pulses with a pulse width below 1 ms, in particular below 0,5 ms and preferably about 0,1 ms, or to emit a combination of the above signals.

The DC current application is believed to produce wound healing, and the pulse signals, when applied directly into the wounds and not applied to a hand or to another distal place as known in the prior art, e.g. by TENS treatments, have been discovered to produce a very good pain-relief effect.

Thus a system is provided for wound treatment, which system is so simple and safe to use that it may be possible to let a patient administer the system himself, possibly in his home. The system provides effective pain relief and healing of the wounds. The treatment does not involve any risk of skin irritation. There is a choice between various functions so that the system may be used exclusively for pain relief, exclusively for healing or for a combination of these, depending upon the particular electric signal emitted by the generator.

The material for forming the surface contact with the wound surface or with a skin surface may be any skin-compatible electrically conductive material capable of conforming to the surface so as to produce an essentially uninterrupted surface contact.

According to one particular embodiment this material may comprise electrically conductive foam plastic.

According to a more preferred embodiment the electrically conductive material comprises a hydrophilic electrically conductive gel capable of absorbing liquid by swelling. The viscous and liquid absorbent properties ensure an excellent and endurable surface contact even by various kinds of oozing wounds. These compresses may be left on the skin for relatively extended time intervals, e.g. several days, which means that the system needs only limited attention, is cheap in use and does not cause any substantial discomfort for a patient.

A particularly advantageous gel is the gel disclosed in the above-mentioned US patents nos. 4 684 558 and 4 706 680. This gel is very sticky, but may still be peeled off the skin when removal is desired without any discomfort and without leaving any residue. This gel is water absorbent up to three times its own weight and with no substantial deterioration of its adhesive properties or conductivity properties. The gel has a substantial heat capacity providing some protection against thermal shocks where it is applied. The gel further exhibits excellent skin compatibility.

This kind of electrode may be manufactured so cheaply that it may be discarded after use, which is advantageous with regard to sterility considerations.

The return electrode may be any kind of electrode known in the art. The return electrode may more particularly be formed similarly to any of the kinds of wound electrode compresses discussed above. The return electrode and the wound electrode within a system may be similar or may be dissimilar.

The invention further provides an electric generator for a wound treatment system, said generator being characterized by being adapted to emit a direct current signal and a superimposed pulsed signal comprising pulses with a pulse width below 1 ms, in particular below 0,5 ms and preferably around 0,1 ms, or to emit a combination of the signals mentioned. This generator is capable of a variety of functions in that the treatment may be solely pain relieving, solely healing or it may be pain relieving and healing. The generator is preferably battery powered to make its use more comfortable for a patient.

According to a preferred embodiment of the generator it is adapted to emit rhythmic trains of electric pulses superimposed onto a direct current signal. Hereby stimulation of the blood flow in the extremities of the body may be obtained with the related well-known advantageous effects.

According to a preferred embodiment the generator is adapted to maintain the levels of the emitted currents essentially constant and independent of the impedances of the electric loads connected to the generator. It is hereby obtained that the treatment signal is well defined and not very sensitive to circumstances, e.g. variations in the gel water content or variations in the implacement of the electrodes onto the body.

The invention further provides a compress for the use by the treatment of wounds by electric stimulation, said compress being characterized by comprising a laminate with a first layer of electrically conductive material capable of forming an essentially uninterrupted surface contact with a wound surface or a skin surface and a second layer of electrically conductive electrode material, said second layer of material exhibiting an electric conductivity in directions parallel to the planes of the layers which is higher than the conductivity of the first layer, and said second layer being provided with means for the attachment of an electric connection.

The compresses for application over the wound or for the return electrode may be retained onto the skin surface by the gel adhesion, by separate plasters, by dressings or by any other prior art means. According to a preferred embodiment the wound compress and/or the return electrode compress comprises a further backing layer with a marginal region projecting beyond the other layers, said marginal region being covered with skin adhesive material so that the compress may be adhered to the skin with no other attachment means.

According to a preferred embodiment the second electrically conductive layer of the compress comprises a conductive polymere film or a synthetic material, e.g. silicone rubber, which has been made electrically conductive by the admixing of carbon. Hereby a better skin compatibility, i.e. a smaller risk of skin irritation, is achieved. The composition of these materials is a critical factor as some electrochemical migration of ions from the compress into the skin is inevitable during the electric stimulation.

The invention further provides a combination of compresses for the treatment of wounds by electric stimulation, said combination comprising a wound compress and a return electrode compress, the electric conductivity of the wound compress in the direction perpendicular to the skin contact surface being lower than the corresponding value for the return electrode compress. A comparatively high conductivity in the return electrode compress is advantageous in order that the compress area does not need to be so large, in order to conserve eletric energy and in order to avoid undesirable heat dissipation. On the other hand, it is advantageous to keep the conductivity of the wound compress comparatively lower in order to ensure a uniform current intensity over the surface of the wound and the surrounding skin. As the conductivity properties of the wound and of the skin may vary over the surface, there may be a risk of concentration of a current within portions of the area so that the current intensity will not be uniform all over the surface, and the current stimulation deviates from the desired values. A comparatively low conductivity of the wound compress is advantageous as it tends to equalize the current density over the contact surface.

In a further preferred embodiment the invention provides a combined compress integrating the wound electrode and the return electrode into a one-piece element advantageously by providing above a common electrically conductive skin contact layer an electrically conductive electrode layer divided into at least two regions with no direct electric interconnection, each region being provided with a respective electric connection. In this compress some leakage current may naturally pass through the electrically conductive skin contact layer from one of the said regions to the other one.

This leakage current may, however, be kept below a suitably moderate level provided that the electric conductivity of the skin contact layer is suitably low and the distance between the two electrode regions is suitably high. The inventor has discovered that with this compress, wherein the wound electrode and the return electrode are arranged close to each other, sufficient electric stimulation of the tissue below the skin where the compress is applied may be induced to produce the above-mentioned advantageous effects. This embodiment is obviously simpler to manufacture, to stock and to market as well as to use as only one piece of compress needs to be handled instead of two, as required in other embodiments.

According to a preferred embodiment the generator is adapted to emit a signal comprising a train of pulses with a frequency of repetition about 100 Hz over a period of time ranging from 40 ms to 400 ms, and preferably around 250 ms, followed by a pulse intermission whereafter the sequence is repeated corresponding to a frequency of pulse train repetition about 2 Hz. Practical testing has proved that this signal provides a particularly good stimulation of the blood flow in the body extremities.

According to a preferred embodiment the generator is programmed for automatically switching over after the expiry of a first predetermined interval of time from the emission of a continuous pulsed signal, possibly superimposed onto a direct current signal, to rhythmic pulse trains, possibly superimposed onto a direct current signal, and after the expiry of a second predetermined interval of time to emit a direct current signal alone. This provides a simpler operation and improves the certainty for the treatment as the generator automatically ensures that a pain relieving, a blood-flow stimulating and a healing signal is emit ted. Furthermore this improves the safety that undesirable side effects, e.g. induced headache, are not produced.

According to a further aspect the invention provides a method for pain-relieving and/or healing treatment of wounds for a patient by means of electric stimulation, wherein an electrically conductive wound compress is placed in contact with the wound and an electrically conductive return electrode compress is placed onto the skin at a distance from the wound, said method being characterized by the application onto the two electrically conductive compresses of an electric signal comprising a combination of a direct current signal and an alternating current signal, e.g. a pulse signal. In practicing this method any of the above-mentioned wound treatment systems, compresses and generators may be used.

Brief Description of the Drawings

Further features, advantages and objects of the invention will appear from the following detailed description of preferred embodiments given with reference to the accompanying drawings, wherein

figure 1 is an overall perspective view of a wound treatment system according to the invention,

figure 2 shows a wound treatment compress according to the invention in a planar view as seen from the skin contact side,

figure 3 shows a wound treatment compress in a sectional view, the section taken along the line 3-3 in figure 2,

figure 4 shows a wound treatment compress according to another embodiment in a sectional view similar to the view in figure 3,

figure 5 is a graphical plot showing the stimulation signal versus time in large scale,

figures 6a, 6b and 6c are graphical plots to a smaller scale than figure 5, figure 6a showing a periodic pulse signal, figure 6b showing a rhythmic pulse signal and figure 6c showing a direct current signal,

figure 7 is an overall perspective view of a wound treatment system according to another embodiment of the invention, and

figure 8 is a sectional view through a wound treatment compress according to the embodiment of figure 7.

## DESCRIPTION OF THE PREFERRED EMBODIMENTS

Description of the Drawings

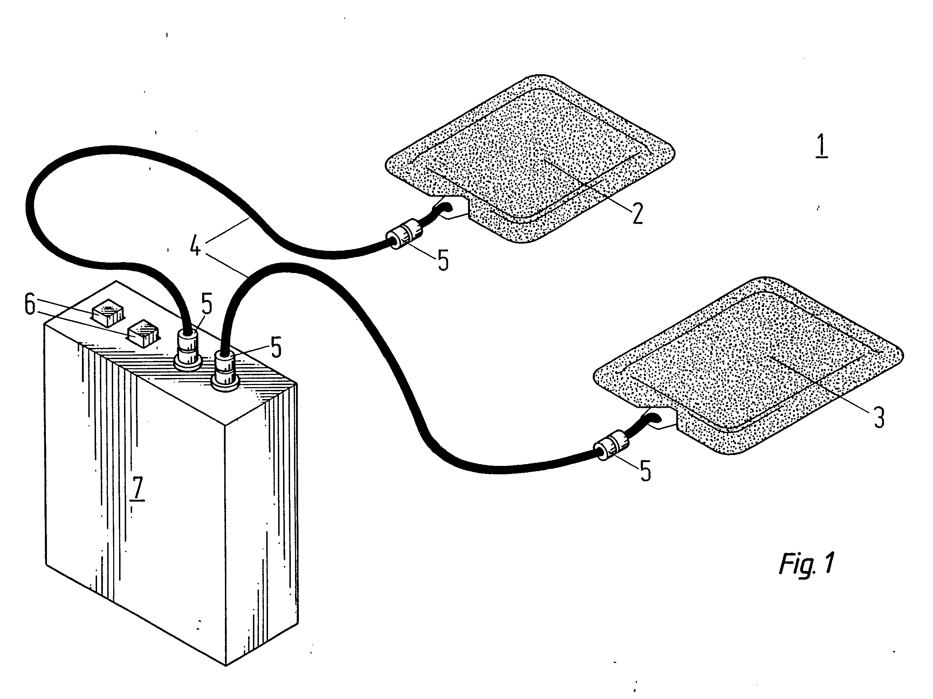

Reference is first made to figure 1 showing a wound treatment system generally indicated by the reference numeral 1, a wound compress or wound plaster 2, a return electrode compress or return electrode plaster 3, an electric generator 7, cables 4 to connect the respective compresses to the generator and cable connectors 5 so that the individual parts of the system may be connected or disconnected in a simple manner. The electric generator 7 is provided with operation buttons 6 so that the operation may be started, stopped and controlled.

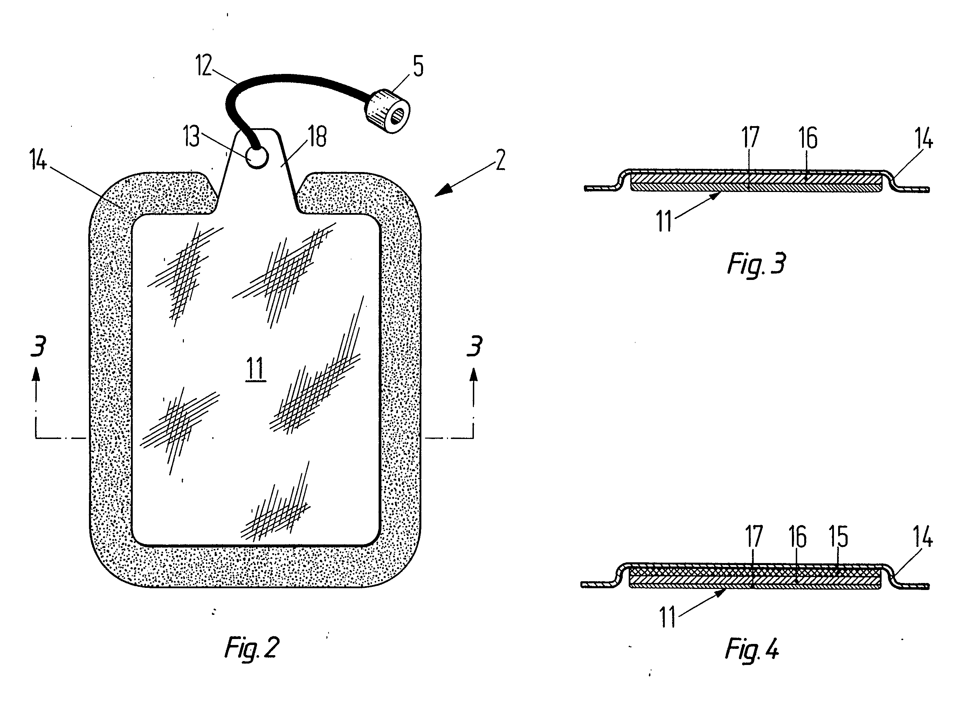

As indicated in figure 2 the wound treatment plaster comprises an electrically conductive skin contact surface 11 essentially rectangular and with a projecting tab 18, to which a cable 12 has been connected at the junction point 13. The electrically conductive contact surface 11 is surrounded by the marginal region of a layer of gaze 14 covered with the skin compatible adhesive intended to retain the plaster onto the skin.

The structure of the wound plaster appears more clearly from the sectional view in figure 3. The bottom layer intended to contact the skin comprises an electrically conductive skin contact layer 17 with a thickness, e.g. about 2 mm, and this layer may e.g. comprise a hydrophilic gel. The gel may be manufactured as described in the above-referenced US patent no. 4 684 558 from an aqueous solution of linear water-soluble polyethylene oxide wherein the polyethylene oxide has been crosslinked by radiation. This gel may have been made electrically conductive by the admixing of a metal salt, in particular an alkali metal salt and preferably natrium chloride or potassium chloride. The gel may contain 93-96% water fully bound in the structure. This gel is capable of absorbing water up to two to three times its own weight. The salt content in the gel may be in the range of 0,1 to 15%, e.g. 0,7 to 10% and preferably 3 to 5% dependent upon the conductivity desired, the conductivity being correlated with the salt content so that an increased salt percentage increases the conductivity.

For the wound treatment plaster salt concentrations towards the lower ends of the above ranges are preferred in order to obtain a compa ratively low conductivity. A high conductivity of the wound plaster is undesirable as the current density may then vary too much in cases where the skin or the wound exhibits varying conductivity over the surface. A comparatively low conductivity in the wound plaster's skin contact layer will tend to smoothen out the current density over the surface area. The gel is viscous and capable of flowing into the smallest interstices in the skin where applied to form an intimate contact with the skin surface.

The layer following the layer 17 is an electrically highly conductive electrode layer, e.g. an electrically conductive foil 16. This foil is in intimate, mechanical and electrical contact with the layer 17 and serves to conduct the current in directions parallel to the plane of the plaster. The electrically conductive foil 16 may comprise a net or a foil of metal, such as tin and preferably tin with a purity rating about 99,93%. According to another embodiment the electrically conductive layer 16 comprises a conductive polymere film. According to a third preferred embodiment the electrically conductive layer comprises synthetic material such as silicone rubber with a thickness about 1 mm, which has been made electrically conductive by the admixing of carbon.

According to a particularly preferred embodiment the electrically conductive layer 16 comprises carbon fiber fabric.

Carbon is considered particularly advantageous as carbon is non-irritating to the skin also when introduced into the skin by the inevitable electrochemical migration developed under the electrodes due to the application of electric current over extended periods of time. The electrically conductive foil 16 projects to form the tab 18 whereby it is connected to the cable 12 at the junction point 13 (cf. figure 2).

On top of the electrically conductive foil a backing layer 14 is provided of a material, e.g. conventional gaze, with a skin compatible adhesive along the margin and with a thickness about 0,2 mm. The marginal region of this layer projects beyond the contour of the other layers so that it may serve to retain the wound plaster along the edge.

If it is desirable to retain the compress in other ways, e.g. to avoid skin irritation by the adhesive, a user may simply cut away the marginal region, or the compress may be supplied in a version without this region. Figure 4 shows a sectional view similar to figure 3, but of another embodiment of the wound treatment compress. The embodiment of figure 4 comprises components similar to all of the components of the embodiment according to figure 3, but differs by comprising an additional reinforcement layer 15 arranged between the electrically conductive foil 16 and the backing layer 14.

The return electrode 3, which is indicated in figure 1, may be an electrode plaster similar to any of the wound plasters explained above, so reference regarding the return electrode may be had to the above-given explanation and to figures 2, 3 and 4. The various types of electrode plasters may be freely combined, the wound plaster and the return electrode plaster within one wound treatment system not needing to be similar.

It is advantageous to use a return electrode plaster with a gel having a somewhat higher conductivity than that of the wound treatment plaster. This may be obtained by a suitable choice of the salt concentration in the gel. The salt concentration is within the ranges mentioned above, but towards the higher ends of the ranges, in order to provide the return electrode plaster with a comparatively high electrical conductivity, e.g. 1,5 to 2 times the wound plaster conductivity.

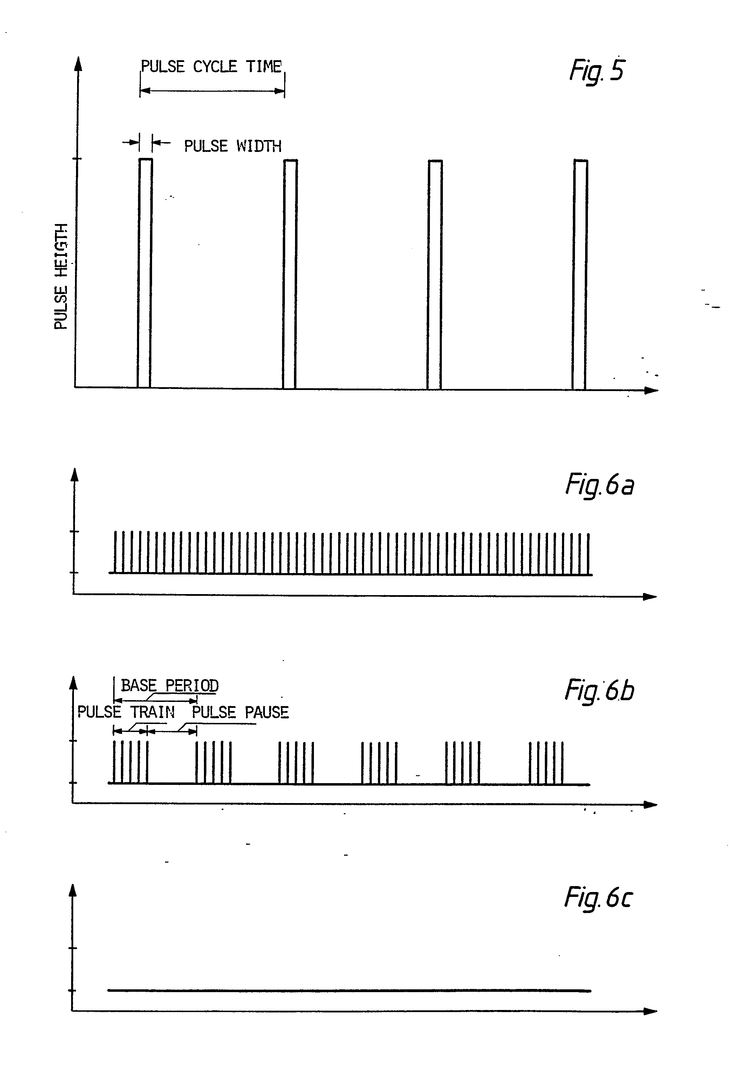

The electrical generator 7 is shown in figure 1 as a portable battery powered device and it is as mentioned above provided with suitable operation buttons 6, whereby it may be switched on, switched off and set to suitable modes of function. The generator is provided with connectors for two cables so that the wound plaster and the return electrode plaster may be connected to the respective connectors. In a first mode of operation the generator emits periodic pulses as illustrated in the plot in figure 5. The width of the individual pulses is below 1 ms, in particular below 0,5 ms and preferably about 0,1 ms. The pulses are emitted periodically, the cyclus time from one pulse to the consecutive pulse being within the range of 2-100 ms, in particular between 5-20 ms and preferably about 10 ms corresponding to a pulse frequency of 100 Hz. The pulse amplitude is defined by an adjustable current value, which may be adjusted within the range of 0-60 mA, the selected current level being maintained essentially independently of the load, except that the output voltage is limited so as not to exceed a maximum of 150 V.

Whereas the generator in a first mode of operation emits periodic pulses, it may be switched to a second mode of operation to emit a train of pulses over a period of time ranging from 40-400 ms, in particular from 100-300 ms and preferably about 250 ms, followed by a pulse intermission and then repeating this sequence at a pulse train base frequency between 1-5 Hz, and preferably about 2 Hz. This mode of operation is designated rhythmic pulses. In a preferred embodiment, wherein the pulse repetition rate is 100 Hz over a period of 200 ms, 25 pulses will be emitted, and assuming a pulse train base frequency of 2 Hz the intermission will last 250 ms.

In a third mode of operation the generator emits a direct current signal as indicated in figure 6c, said signal being maintained at a predetermined level of current essentially independent of the load, e.g. at 800 $\mu$A, except that the output voltage is limited to not exceed 9 V. Assuming a wound plaster contact area of 83 cm², the current density over this area will be about 10 $\mu$A per cm².

The above-explained modes of operation may be combined as illustrated in figures 6a and 6b, e.g. to emit as illustrated in figure 6a periodic pulses superimposed onto the direct current signal, or the generator may emit the rhythmic signal illustrated in figure 6b comprising pulse trains with intermediate pauses and superimposed onto the direct current signal.

The effects of the various stimulation signals are believed to be so that the periodic signal primarily is pain relieving, that the rhythmic signal in particular improves the blood flow in the body extremities and that the direct current signal in particular enhances the wound healing.

According to a preferred embodiment the generator is preprogrammed so that it, upon being switched on, initially emits the signal of figure 6a over a first predetermined interval of time, e.g. 20-25 minutes, thereafter switching automatically to emit the signal shown in figure 6b, which is emitted over a second predetermined interval of time, e.g.

20-25 minutes, then switching automatically to emit the signal of figure 6c, said signal being emitted for a third predetermined interval of time, e.g. 60-120 minutes, whereafter the generator is automatically turned off. Practical experience has shown that this programme ensures a good pain-relieving effect, a good effect onto the blood flow and a good healing effect at a minimum consumption of electric power so that the batteries in the generator are preserved as much as possible. The generator may hereby be constructed comparatively small in order to be easily portable and the service life of the batteries is extended.

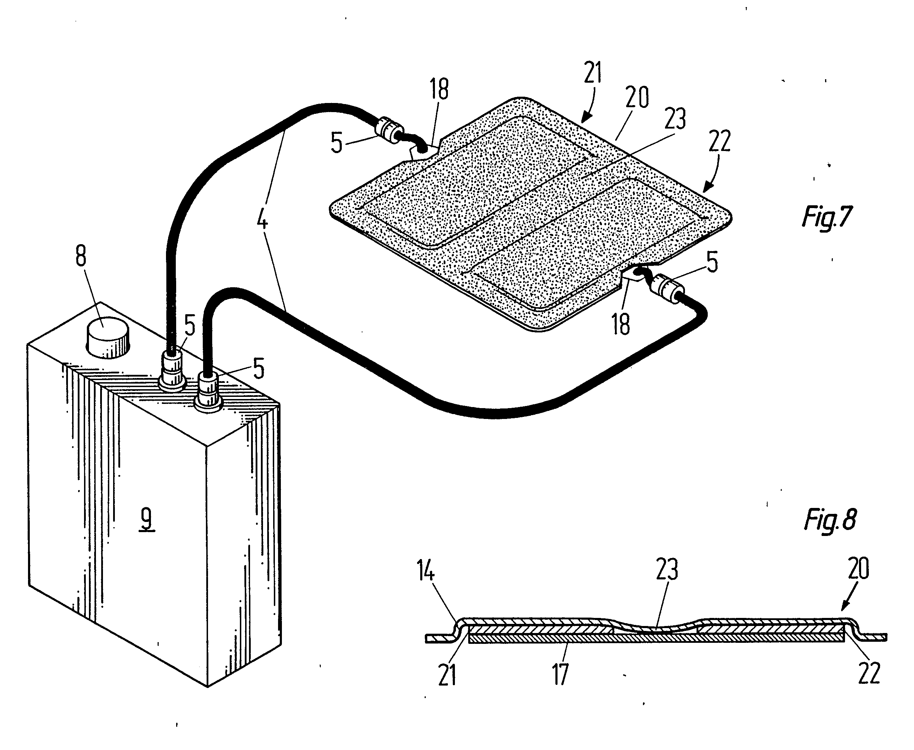

Reference is now made to figures 7 and 8 wherein another advantageous embodiment of the invention is shown. Some of the elements of this embodiment are similar to those of the embodiments described above and therefore referenced with the same reference numerals. Figure 7 shows a wound treatment system comprising a generator 9 and a double compress 20, these parts being interconnected by suitable cables 4 and connectors 5 similarly as the other embodiments.

As clearly illustrated in the sectional view of figure 8 the double compress correspondingly as the compress of figure 3 comprises a skin contact layer 17 and a backing layer 14. The embodiment of figures 7 and 8 differs by the electrically conductive electrode layer being divided into two regions, a wound electrode region 21 and a return electrode region 22, respectively. These two regions are separated by a demarcation region 23 with no electrically conductive electrode layer so that the distance between the closest parts of the two electrode regions is everywhere above a predetermined lower limit, e.g. 10 mm. As evident from figure 7 the wound electrode region 21 and the return electrode region 22 is each provided with a respective electrical connection tab 18 and they may be used as a wound electrode and a return electrode, respectively.

Since the skin contact layer 17 has some electric conductivity, some leakage current may obviously pass the skin contact layer 17 in the direction parallel to the front surface and across the demarcation region 23. However, this leakage current may be kept below an acceptable level provided that the conductivity of the skin contact layer 17 is suitably low and provided that the width of the demarcation region 23 is everywhere at least 10 mm. The inventor has discovered that the current stimulus signal by this double compress surprisingly penetrates sufficiently far into the tissues below the skin to induce the same advantageous effects as explained above.

With this embodiment a simpler handling and application of the compress is obtained as only one piece of compress needs to be applied. The manufacture of the compress is also simplified in particular for the versions where the materials within the wound electrode and the return electrode are completely similar. Although the two regions of the double compress have been designated wound electrode and return electrode, respectively, they may obviously be made completely alike and thus interchangeable relative to the wound. Thus the double compress may be used with either one of the electrode regions placed over the wounds or may be used with the demarcation region placed over the wound or with a com bination of the two electrode regions and the demarcation region placed over the wound.

The generator 9 shown in figure 7 is provided with a turnable button 8 for the control. The button 8 has been designed so that the generator is turned off by turning the button all the way forwards a stop to one of the sides. When the generator is to be used the button is turned away from the turned-off position whereby the generator is switched on automatically commencing its preprogrammed function emitting pulses at a level depending upon the excursion of the turn of the button. The operator slowly turns the button 8 further in the same direction whereby the intensity of the pulse signals increases. At some level a patient can feel the pulses and the button may be turned as far as acceptable so as not to exceed the comfortable limits. The generator follows its preprogrammed switching as explained above with reference to the embodiment shown in figure 1.

The generator may be turned off at any time by turning the button into the switched off position.

### Example

A patient suffering painful chronic wounds on both tibiae was insensitive to all known types of treatment and amputation was under consideration.

This patient was treated according to the invention, a wound electrode with a hydrophilic gel being applied over the wound on the right tibia and the return electrode being applied onto the opposite side of the same tibia. An electric generator according to the invention was connected to the electrode.

Electric stimulation was administered twice daily and each time so as to stimulate initially 20 minutes with continuous pulses superimposed onto a direct current signal, thereafter 20 minutes with the rhythmic pulses superimposed onto a direct current signal and finally 1 hour and 20 minutes with a direct current signal. The polarity of the generator electrodes was reversed once daily. The plasters were replaced once every three days.

The patient reported a good pain-relieving effect after just a few minutes of stimulation. After three days initial healing of the wound on the right tibia was observed. After two weeks of treatment the wound on the right leg was completely healed with a full epithelization whereas some healing of the wound on the left tibia was also observed. The initial healing of the wound on the left tibia is believed to be due to the general enhancement of the blood flow developed by the rhythmic pulse signal.

While in the foregoing an embodiment of the invention has been disclosed in considerable detail for purposes of illustration, it will be understood by a person skilled in the art that may of these details may be varied without departing from the spirit and scope of the invention.

### Claims

1. A wound treatment system comprising an electrically conductive wound compress, a return electrode, an electric generator and means, e.g. cables, for interconnecting the above parts, **characterized**
by the wound compress comprising a laminate with a first layer of electrically conductive material capable of forming an essentially uninterrupted surface contact with a wound surface or with a skin surface and a second layer of electrically conductive electrode material, said second layer exhibiting a conductivity in directions parallel to the planes of the layers which is higher than the corresponding value for the first layer, said second layer being adapted for attachment of an electric connection, and
by the generator being adapted to optionally emit a direct current signal or a pulse signal comprising pulses with a pulse duration below 1 ms, in particular below 0,5 ms and preferably around 0,1 ms, or a combination of the above signals, said signals being limited to stay below a level where they are personally safe.

2. The wound treatment system of claim 1, **characterized** by the wound compress first layer comprising an electrically conductive hydrophilic gel formed by crosslinking linear polyethylene oxide dissolved in water.

3. An electric generator for a wound treatment system, **characterized** by being adapted to emit a direct current signal superimposed onto a pulsed signal comprising pulses with a pulse duration below 1 ms, in particular below 0,5 ms and preferably about 0,1 ms, or a combination of the above signals, said signals being limited to stay below a level where they are personally safe, by said generator being adapted to repeat the pulsed signal periodically with a cycle frequency within the range of 20-500 Hz, in particular from 50-200 Hz and preferably about 100 Hz, and being adapted to emit the pulsed signal cyclically to form a pulse train over a range of time lasting from 40-400 ms, in particular from 100-300 ms and preferably about 250 ms, followed by an intermission whereafter this sequence is repeated, the repetition rate of pulse trains forming a base frequency between 1 and 5 Hz, and preferably about 2 Hz.

4. The generator of claim 3, **characterized** by being adapted to automatically switch over its mode of operation after a first predetermined interval of time from emitting a continuous pulse signal superimposed onto the direct current signal to emit periodic pulse trains superimposed onto the direct current signal and to switch over after a second predetermined interval of time to emit a direct current signal.

5. A compress for treatment of wounds by means of electric stimulation, **characterized** by comprising a laminate with a first layer of electrically conductive material capable of forming an essentially uninterrupted surface contact with a wound surface or with a skin surface and a second layer of electrically conductive electrode material, said second layer exhibiting a conductivity in directions parallel to the planes of the layers which is higher than the corresponding value for the first layer, said second layer being adapted for attachment of an electric connection.

6. A compress for the treatment of wounds by means of electric stimulation, **characterized** by comprising a laminate with a first layer of electrically conductive material capable of forming an essentially uninterrupted surface contact with a wound surface or with a skin surface and a second layer of electrically conductive electrode material, said second layer exhibiting a conductivity in directions parallel to the planes of the layers which is higher than the corresponding value for the first layer, said second layer being divided into at least two regions with no direct electric interconnection, each of the two regions being adapted for attachment of a respective electric connection.

7. The compress of claim 5 or 6, **characterized** by said first layer comprising an electrically conductive hydrophilic gel formed by cross-linking of linear polyethylene oxide dissolved in water.

8. The compress of any of the claims 5 through 7, **characterized** by said second layer comprising a carbon fiber fabric.

9. A combination of compresses for the treatment of wounds by means of electric stimulation comprising a wound compress intended for fitting over or adjacent the wound site and a return electrode compress intended for fitting distally from the wound site, **characterized** by said wound compress comprising a laminate with a first layer of electrically conductive material capable of forming an essentially uninterrupted surface contact with a wound surface or with a skin surface and a second layer of electrically conductive electrode material, said second layer exhibiting a conductivity in directions parallel to the planes of the layers which is higher than the corresponding value for the first layer, said second layer being adapted for attachment of an electric connection, and by the wound compress first layer exhibiting an electric conductivity in directions perpendicular to the surface which is lower than the corresponding value of the return electrode compress first layer.

10. The combination of compresses of claim 9, **characterized** by the return electrode compress first layer exhibiting a conductivity in directions perpendicular to the contact surface which is 1,5 to 2 times the conductivity of the wound compress first layer in directions perpendicular to the contact surface.

Fig. 1

EP 0 367 320 A1

Fig. 2

Fig. 3

Fig. 4

EP 0 367 320 A1

EP 0 367 320 A1

Fig. 5

PULSE CYCLE TIME

PULSE WIDTH

PULSE HEIGTH

Fig. 6a

Fig. 6b

BASE PERIOD

PULSE TRAIN   PULSE PAUSE

Fig. 6c

Fig.7

Fig.8

EP 0 367 320 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.X)5 |
|---|---|---|---|
| D,Y | US - A - 4 706 680 (KEUSCH) | 1 | A 61 N 1/04 A 61 N 1/36 |
| X | * Abstract; fig. 1-11; column 6, lines 3-66 * | 2,5-7, 9,10 | |
| Y | US - A - 4 520 825 (THOMPSON) | 1 | |
| X | * Abstract; column 2, lines 5-20; column 5, lines 4-17 * | 3,4 | |
| P,A | FR - A1 - 2 614 792 (MONTANE) | 5,6 | |
| X | * Abstract; claim 1; fig. 1,2 * | 8 | |
| A | EP - A1 - 0 223 340 (KUREHA) * Abstract; fig. 1 * | 1,5-10 | |
| Y | DE - A1 - 2 552 197 (KAUFMAN) | 1 | TECHNICAL FIELDS SEARCHED (Int. Cl.X 5) A 61 N A 61 B |
| X | * Claim 1; fig. 1,2 * | 5,6,9, 10 | |
| Y | US - A - 4 535 777 (CASTEL) | 1 | |
| X | * Abstract; column 1, lines 14-43; column 2, lines 1-42; fig. 1a-1d * | 3,4 | |
| A | AT - B - 376 129 (BARD) * Fig. 1,2,4 * | 1,5,6, 9,10 | |

The present search report has been drawn up for all claims

| Place of search | Date of completion of the search | Examiner |
|---|---|---|
| VIENNA | 29-01-1990 | NEGWER |